# EUROPEAN PATENT SPECIFICATION

(11) **EP 1 482 955 B1**
(45) Date of publication and mention of the grant of the patent: **25.12.2013**
(21) Application number: 02724914.3
(22) Date of filing: 04.02.2002
(51) Int. Cl.: A61K 31/70, A61P 37/04, A61P 37/08, A61P 31/00

(54) **PROPHYLACTIC AND THERAPEUTIC TREATMENT OF INFECTIOUS AND OTHER DISEASES WITH IMMUNOEFFECTOR COMPOUNDS**
PROPHYLAKTISCHE UND THERAPEUTISCHE BEHANDLUNG VON INFEKTIONS- UND ANDEREN KRANKHEITEN MIT IMMUNOEFFEKTOR-VERBINDUNGEN
TRAITEMENT PROPHYLACTIQUE ET THERAPEUTIQUE DE MALADIES INFECTIEUSES ET AUTRES MALADIES UTILISANT DES COMPOSES IMMUNO-EFFECTEURS

(43) Date of publication of application: 08.12.2004
(73) Proprietor: CORIXA CORPORATION, Seattle, WA 98104 (US)
(72) Inventor: BALDRIDGE, Jory, R., Victor, MT 59875 (US); JOHNSON, David, A., Hamilton, MT 59840 (US); CLUFF, Christopher, W., Hamilton, MT 59840 (US)
(74) Representative: Harrison Goddard Foote LLP
(86) International application number: PCT/US2002/003581
(87) International publication number: WO 2003/066065

(56) References cited:
- WO-A-02/12258
- WO-A-03/065806
- US-A- 6 113 918

## Description

### BACKGROUND OF THE INVENTION

The innate immune system coordinates the inflammatory response to pathogens by a system that discriminates between self and non-self via receptors that identify classes of molecules synthesized exclusively by microbes. These classes are sometimes referred to as pathogen associated molecular patterns (PAMPs) and include, for example, lipopolysaccharide (LPS), peptidoglycans, lipotechoic acids, and bacterial lipoproteins (BLPs).

LPS, an abundant outer cell-wall constituent from gram-negative bacteria, is recognized by the innate immune system. Although the chemical structure of LPS has been known for some time, the molecular basis of recognition of LPS by serum proteins and/or cells is only now being elucidated. In a series of recent reports, a family of receptors, referred to as Toll-like receptors (TLRs), have been linked to the potent innate immune response to LPS and other microbial components. TLR are membrane proteins having a single transmembrane domain. The cytoplasmic domains are approximately 200 amino acids and share similarity with the cytoplasmic domain of the IL-1 receptor. The extracellular domains are relatively large (about 550-980 amino acids) and may contain multiple ligand-binding sites.

The importance of TLRs in the immune response to LP9 has been specifically demonstrated for at least two Toll-like receptors, Tlr2 and Tlr4. For example, transfection studies with embryonic kidney cells revealed that human Tlr2 was sufficient to confer responsiveness to LPS (Yang et al., Nature 395:284-288 (1998); Kirschning et al. J Exp Med. 11:2091-97 (1998)). A strong response by LPS appeared to require both the LPS-binding protein (LBP) and CD14, which binds LPS with high affinity. Direct binding of LPS to Tlr2 was observed at a relatively low affinity, suggesting that accessory proteins may facilitate binding and/or activation of Tlr2 by LPS in vivo.

The importance of Tlr4 in the immune response to LPS was demonstrated in conjunction with positional cloning in *lps* mutant mouse strains. Two mutant alleles of the mouse *lps* gene have been identified, a semidominant allele that arose in the C3H/HeJ strain and a second, recessive allele that is present in the C57BL/10ScN and C57BL/10ScCr strains. Mice that are homozygous for mutant alleles of *lps* are sensitive to infection by Gram-negative bacteria and are resistant to LPS-induced septic shock. The *lps* locus from these strains was cloned and it was demonstrated that the mutations altered the mouse Tlr4 gene in both instances (Portorak et al., Science 282:2085-2088 (1998); Qureshi et al., J Exp Med 4:615-625 (1999)). It was concluded from these reports that Tlr4 was required for a response to LPS.

The biologically active endotoxic sub-structural moiety of LPS is lipid-A, a phosphorylated, multiply fatty-acid-acylated glucosamine disaccharide that serves to anchor the entire structure in they outer membrane of Gram-negative bacteria. We previously reported that the toxic effects of lipid A could be ameliorated by selective chemical modification of lipid A to produce monophosphoryl lipid A compounds (MPL® immunostimulant; Corixa Corporation; Seattle, WA). Methods of making and using MPL® immunostimulant, and structurally like compounds, for vaccine adjuvant and other applications have been described (see, for example, U.S. Patent Nos. 4,436,727; 4,877,611; 4,866,034 and 4,912,094; 4,987,237; Johnson et al., J Med Chem 42:4640-4649 (1999); Ulrich and Myers, in Vaccine Design: The Subunit and Adjuvant Approach; Powell and Newman, Eds.; Plenum: New York, 495-524, 1995). In particular, these and other references demonstrated that MPL^{®} immunostimulant and related compounds had significant adjuvant activities for enhancing humoral and/or cell-mediated immunity to the antigens, when used in vaccine formulations with protein and carbohydrate antigens.

Synthetic mono- and disaccharide molecules which share structural similarities with MPL^{®} immunostimulant, referred to as aminoalkyl glucosaminide phosphates (AGPs), have been described, see for example, U.S. Patent No. 6,113,918, U.S. Patent No. 6,303,347, and WO 98/50399, published October 12, 1998. These compounds retain significant adjuvant characteristics when formulated with antigens in vaccine compositions and have similar or improved toxicity profiles when compared with monophosphoryl lipid A. These compounds have been described for use in combination with antigens in vaccine formulations (U.S. Patent No. 6,113,918) and in the absence of antigen, as monotherapies, WO 01/90129, published 29-Nov-2001.

Cyclic aminoalkyl glucosaminide phosphates or "cyclic AGPs" have been described in PCT Patent Application No. PCT/US01/24284 (WO 02/12258). These cyclic AGPs are effective immunoeffector molecules which enhance humoral and cell-mediated immune responses to vaccine antigens. As used herein, the term "cyclic AGP" means an azacycloalkyl or (azacycloalkyl)alkyl glucosaminide phosphate, wherein a 2-deoxy-2-amino-b-D-glucopyranose (glucosamine) is glycosidically linked to an azacycloalkyl or (azacycloalkyl)alkyl (aglycon) group.

The present invention provides cyclic AGPs for use as monotherapies formulated and administered in the absence of exogenous antigens for the prophylactic and/or therapeutic treatment of plant and animal diseases and conditions, such as infectious diseases, autoimmunity and allergies. The monotherapies comprise one or more cyclic AGPs. These and other aspects of the invention will become evident upon reference to the following detailed description and the attached drawings.

### SUMMARY OF THE INVENTION

In one aspect, the present invention provides one or more compounds for use in treating, ameliorating or substantially preventing an infectious disease, autoimmune disease or allergic condition, the one or more compounds having the formula (I): 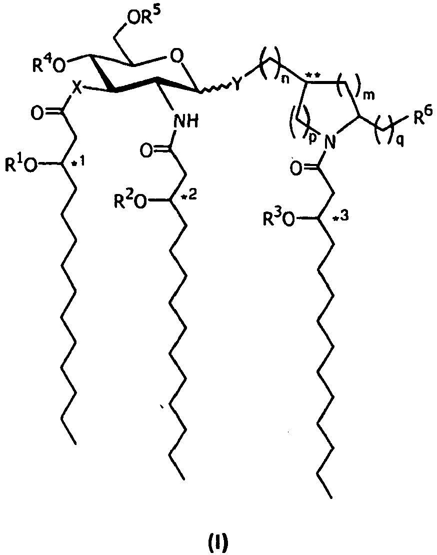 and pharmaceutically acceptable salts thereof, wherein X is -O- or -NH- and Y is -O- or -S-; R¹, R², and R³ are each independently a (C₂-C₂₀)acyl group, including saturated, unsaturated and branched acyl groups; R⁴ is -H or -PO₃R⁷R⁸, wherein R⁷ and R³ are each independently H or (C₁-C₄)aliphatic groups; R⁵ is -H, -CH₃ or -PO₃R⁹R¹⁰, wherein R⁹ and R¹⁰ are each independently selected from -H and (C₁-C₄)aliphatic groups; R⁶ is independently selected from H, OH, (C₁-C₄)oxyaliphatic groups, -PO₃R¹¹R¹², -OPO₃R¹¹R¹², -SO₃R¹¹, - OSO₃R¹¹, -NR¹¹R¹², -SR¹¹, -CN, -NO₂, -CHO, -CO₂R¹¹, and -CONR¹¹R¹², wherein R¹¹ and R¹² are each independently selected from H and (C₁-C₄)aliphatic groups; with the provisos that one of R⁴ and R⁵ is a phosphorus-containing group and that when R⁴ is -PO₃R⁷R⁸, R⁵ is other than -PO₃R⁹R¹⁰, wherein "*¹⁻³" and "**" represent chiral centers; wherein the subscripts n, m, p and q are each independently an integer from 0 to 6, with the proviso that the sum of p and m is from 0 to 6, and wherein the hexapyranoside may be in another configuration than the gluco configuration represented in the formula, selected from allo, altro, manno, gulo, ido, galacto and talo; characterized in that the one or more compounds are administered in the absence of exogenous antigen.

In some embodiments, the compounds of Formula I contain an -O- at X and Y, R⁴ is PO₃R⁷R⁸, R⁵ and R⁶ are H, and the subscripts n, m, p, and q are integers from 0 to 3. In a more preferred embodiment, R⁷ and R⁸ are -H. In one embodiment, subscript n is 1, subscript m is 2, and subscripts p and q are 0. In other embodiments, R₁, R₂, and R₃ are (C₆-C₁₄) acyl, (C₆-C₁₂), or (C₆-C₈)acyl groups, in a particular embodiment are provided (C₆-C₁₂)acyl groups. A further embodiment provides, *¹⁻³ are in the R configuration, Y is in the equatorial position, and ** is in the S configuration.

Illustrative embodiments include *N*-[(*R*)-3-tetradecanoyloxytetradecanoyl]-(*S*)-2-pyrrolidinylmethyl 2-deoXy-4-*O*-phosphono-2-[(R)-3-tetradecanoyloxytetradecanoylamino]-3-*O*-[(*R*)-3-tetradecanoyloxytetradecanoyl]- β-D-glucopyranoside and pharmaceutically acceptable salts thereof; Formula II, 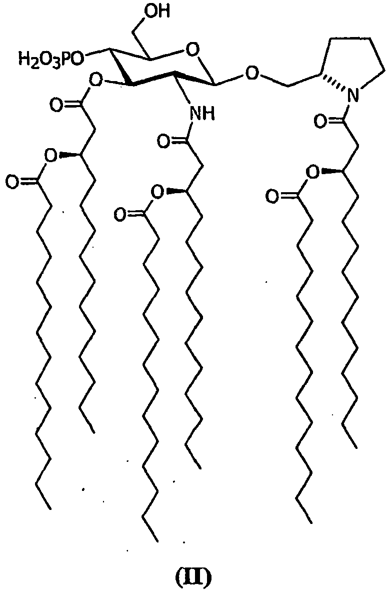 *N*-[(*R*)-3-dodecanoyloxytetradecanoyl]-(*S*)-2-pyrrolidinylmethyl 2-deoxy-4-*O*-phosphono-2-[(*R*)-3-dodecanoyloxytetradecanoylamino]-3-*O*-[(*R*)-3-dodecanoyloxytetradecanoyl]- β-D-glucopyranoside and pharmaceutically acceptable salts thereof; Formula III, 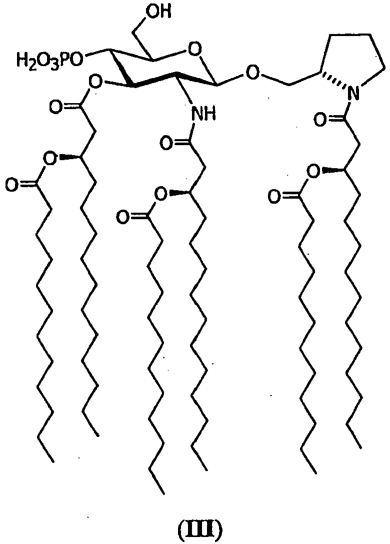 and *N*-[(*R*)-3-decanoyloxytetradecanoyl]-(*S*)-2-pyrrolidinylmethyl 2-deoxy-4-*O*-phosphono-2-[(*R*)-3-decanoyloxytetradecanoylamino]-3-*O*-[(*R*)-3-decanoyloxytetradecanoyl]: β-D-glucopyranoside and pharmaceutically acceptable salts thereof; Formula IV.

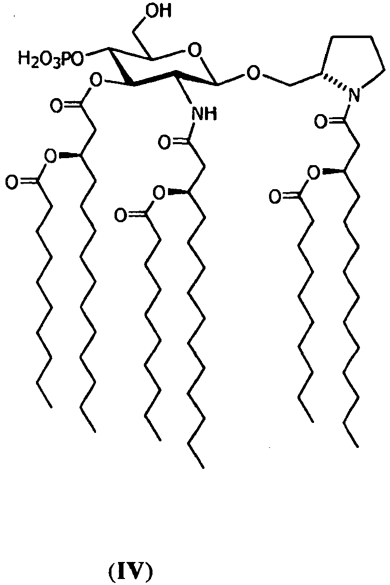

Thus, described herein are methods employed in treating, ameliorating or substantially preventing infectious diseases, autoimmune diseases and allergies.

The present invention, in other aspects, provides a pharmaceutical composition comprising one or more of the compounds described herein in combination with pharmaceutically acceptable carriers or excipients, the composition being for use in treating, ameliorating or substantially preventing an infectious disease, autoimmune disease or allergic condition; characterized in that said composition is formulated in the absence of exogenous antigen and is for administration in the absence of exogenous antigen.

Also provided is a cyclic AGP for use in treating, ameliorating or substantially preventing an infectious disease, autoimmune disease or allergic condition, wherein said cyclic AGP is *N*-[(*R*)-3-dodecanoyloxytetradecanoyl]-(*S*)-2-pyrrolidinylmethyl-2-deoxy-4-*O-*phosphono-2-[(*R*)-3-dodecanoyloxy-tetradecanoylamino]-3-*O*-[(*R*)-3-dodecanoyloxytetradecanoyl]-β-D-glucopyranoside or a pharmaceutically acceptable salt thereof; or *N*-[(*R*)-3-decanoyloxytetradecanoyl]-(*S*)-2-pyrrolidinylmethyl-2-deoxy-4-*O-*phosphono-2-[(*R*)-3-decanoyloxytetradecanoylamino]-3-*O*-[(*R*)-3-decanoyloxytetradecanoyl]-β-D-glucopyranoside or a pharmaceutically acceptable salt thereof;
characterised in that
said cyclic AGP is administered in the absence of exogenous antigen.

### DESCRIPTION OF ILLUSTRATIVE EMBODIMENTS OF THE INVENTION

### ILLUSTRATIVE PROPHYLACTIC AND THERAPEUTIC APPLICATIONS

The present invention broadly concerns one or more compounds described herein or a pharmaceutical composition comprising one or more such compounds for use in prophylactic and therapeutic methods of treating certain diseases and other medical conditions, wherein the treatment is by administration of an effective amount of the one or more compounds or the pharmaceutical composition.

While certain of the cyclic AGP compounds have been described for use as adjuvants in combination with exogenously administered antigens in vaccine formulations, and for use in certain other applications, the present invention describes novel therapeutic uses that employ the compounds preferably in monotherapeutic applications, *i.e*., in the absence of exogenously administered antigen.

In one aspect the present invention provides compounds for use as defined herein wherein said use is for treating, ameliorating and/or substantially preventing infectious diseases in eukaryotic subjects, particularly in animals, preferably in humans. Given the importance of TLR-mediated signalling in the innate immune response to microbial challenge, the ability to stimulate such pathways selectively and with minimal toxicity represents a powerful approach for prophylactic and/or therapeutic treatment modalities against a wide range of infectious agents.

The methods described herein are applicable against essentially any type of infectious agent, including bacteria, viruses, parasites, and fungi. Illustratively, the methods are useful for the prophylactic and/or therapeutic treatment of bacterial infections by species from *Pseudomonas, Escherichia, Klebsiella, Enterobacter, Proteus, Serratia, Candida, Staphylococci, Streptococci, Chlamydia, Mycoplasma, Bacillus, and numerous others.* Illustrative viral conditions that may be treated in accordance with the invention include those caused, for example, by Influenza viruses, Adenoviruses, parainfluenza viruses, Rhinoviruses, respiratory syncytial viruses (RSVs), Herpes viruses, Cytomegaloviruses, Hepatitis viruses, e.g., Hepatitis B and C viruses, and others. Illustrative fungi include, for example, *Aspergillis, Candida albicans, Cryptococcus neoformans, Coccidioides immitus,* and others.

In one illustrative embodiment, the invention provides compounds for use as defined herein wherein said use is for the treatment of subjects, particularly immunocompromised subjects, that have developed or are at risk for developing infections, such as nosocomial bacterial and viral infections. About 2 million of the 40 million individuals hospitalized every year develop nosocomial infection during their stay and about 1% of these, or about 400,000 patients, develop nosocomial pneumonia, more than 7000 of which die. This makes nosocomial pneumonia the leading cause of death in hospital-acquired infections. Thus, this embodiment fills a significant need for effective prophylactic approaches in the treatment of nosocomial infections.

In a related embodiment, the present invention provides compounds for use as defined herein wherein said use is in prophylactic treatments for immunocompromised patients, such as HIV-positive patients, who have developed or are at risk for developing pneumonia from either an opportunistic infection or from the reactivation of a suppressed or latent infection. In 1992, about 20,000 cases of Pneumocystis carinii infections in AIDS patients were reported in the U.S. alone. Additionally, 60-70% of all AIDS patients get P.carinii at some time during their illness. Thus, the compounds for use according to the present invention in this embodiment provide effective prophylactic methods for this at-risk population.

In another related embodiment, the present invention provides compounds for use as defined herein wherein said use is for treating other patient populations that may be immunocompromised and/or at risk for developing infectious diseases, including, for example, patients with cystic fibrosis, chronic obstructive pulmonary disease and other immunocompromized and/or institutionalized patients.

Another aspect of the invention provides compounds for use as described herein wherein said use is for treating, ameliorating or substantially preventing allergic disorders and conditions, such as sinusitis, chronic rhinosinusitus, asthma, atopic dermatitis and psoriasis. This approach is based at least in part on the ability of the compounds to activate the production of cytokines from target cells that can compete with stereotypic allergic-type cytokine responses characterized by IL-4 production or hyperresponsiveness to IL-4 activity. Administration of certain of the compounds disclosed herein results in IFN-gamma and IL-12 expression from antigen processing and presenting cells, as well as other cells, resulting in down regulation of cytokines associated with allergic responses such as IL-4, 5, 6, 10 and 13.

Another aspect of the invention provides compounds for use as defined herein wherein said use is for treating autoimmune diseases and conditions. The compounds for use in this embodiment will typically be selected from those capable of antagonizing, inhibiting or otherwise negatively modulating one or more Toll-like receptors, particularly Tlr2 and/or Tlr4, such that an autoimmune response associated with a given condition is ameliorated or substantially prevented. Illustratively, the compounds for use according to this embodiment can be for use in the treatment of conditions such as inflammatory bowel disease, rheumatoid arthritis, chronic arthritis, multiple sclerosis and psoriasis.

While not wishing to be bound by theory, it is believed that the efficacy of the prophylactic and therapeutic applications described above are based at least in part on the involvement of the compounds in the modulation of Toll-like receptor activity. In particular, Toll-like receptors Tlr2, Tlr4, and others, are believed to be specifically activated, competitively inhibited or otherwise affected by the non-toxic LPS derivatives and mimetics disclosed herein. Accordingly, the invention provides a powerful and selective approach for modulating the innate immune response pathways in animals without giving rise to the toxicities often associated with the native bacterial components that normally stimulate those pathways.

### ILLUSTRATIVE CYCLIC AGP COMPOUNDS

Illustrative compounds employed in the above prophylactic and therapeutic applications comprise compounds of Formula I: 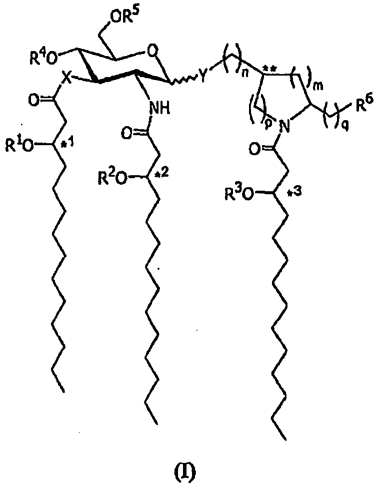 and pharmaceutically acceptable salts thereof, wherein X is -O- or -NH- and Y is -O- or -S- ; R¹, R², and R³ are each independently a (C₂-C₂₀)acyl group, including saturated, unsaturated and branched acyl groups; R⁴ is -H or PO₃R⁷R⁸, wherein R¹ and R⁸ are each independently H or (C₁-C₄)aliphatic groups; R⁵ is -H, -CH₃ or -PO₃R⁹R¹⁰, wherein R⁹ and R¹⁰ are each independently selected from -H and (C₁-C₄)aliphatic groups; R⁶ is independently selected from H, OH, (C₁-C₄)oxyaliphatic groups, -PO₃R¹¹R¹², -OPO₃R¹¹R¹², -SO₃R¹¹, -OSO₃R¹¹, -NR¹¹R¹², -SR¹¹, -CN,-NO₂, -CHO, -CO₂R¹¹, and-CONR¹¹R¹², wherein R¹¹ and R¹² are each independently selected from H and (C₁-C₄)aliphatic groups; with the provisos that one of R⁴ and R⁵ is a phosphorus-containing group and that when R⁴ is - PO₃R⁷R⁸, R⁵ is other than -PO₃R⁹R¹⁰, wherein "*¹⁻³" and "**" represent chiral centers; wherein the subscripts n, m, p and q are each independently an integer from 0 to 6, with the proviso that the sum of p and m is from 0 to 6, and wherein
the hexopyranoside in Formula I may be in another configuration than the gluco configuration represented in the formula, selected from allo, altro, manno, gulo, ido, galacto and talo.

In the general formula above, the configuration of the 3'-stereogenic centers to which the normal fatty acyl residues are attached, denoted "*¹", "*²" and "*³", is *R* or *S*, but preferably *R*. The absolute stereochemistry of the carbon atoms of the cyclic aglycon unit to which R⁶ and the glucosamine unit are attached, directly or indirectly (denoted "**") can be *R* or *S*. In the general formula above, Y can be in the equatorial or axial position, but is preferably equatorial. All stereoisomers, enantiomors, diastereomers and mixtures thereof are considered to be within the scope of the present invention.

In illustrative embodiments of the present invention X and Y are -O-, R⁴ is phosphono, R⁵ and R⁶ are H, and the subscripts n, m, p, and q are integers of from 0 to 3, and more preferably 0 to 2. In an exemplary embodiment the integer n is 1, the integer m is 2, and integers p and q are 0. In this embodiment, the compounds of this invention are 2-pyrrolidinylmethyl β-D-glucosaminide 4-phosphates having the general formula (V):

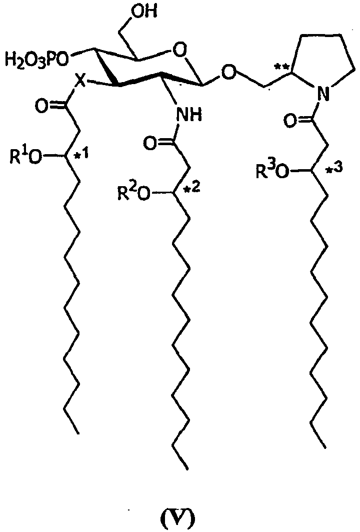

In another illustrative embodiment of the present invention, R₁, R₂, and R₃ of formula (III) are tetradecanoyl residues and the configuration of the 3'-stereogenic centers ("*¹⁻³") to which they are attached is *R*, Y is in the equatorial position, and the absolute stereochemistry of the pyrrolidine stereogenic center ("**") is *S*.

Other exemplary embodiments include *N*-[(*R*)-3-tetradecanoyloxytetradecanoyl]-(*S*)-2-pyrrolidinylmethyl 2-deoxy-4-*O*-phosphono-2-[(*R*)-3-tetradecanoyloxytetradecanoylamino]-3-*O*-[(*R*)-3-tetradecanoyloxytetradecanoyl]-β-D-glucopyranoside, and its pharmaceutically acceptable salts, formula (II): 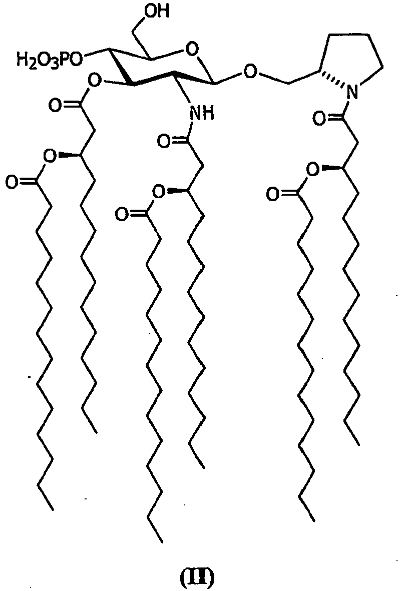 *N*-[(*R*)-3-dodecanoyloxytetradecanoyl]-(*S*)-2-pyrrolidinylmethyl 2-deoxy-4-*O*-phosphono-2-[(*R*)-3-dodecanoyloxytetradecanoylamino]-3-*O*-[(*R*)-3-dodecanoyloxytetradecanoyl]-β-D-glucopyranoside and pharmaceutically acceptable salts thereof; Formula III, 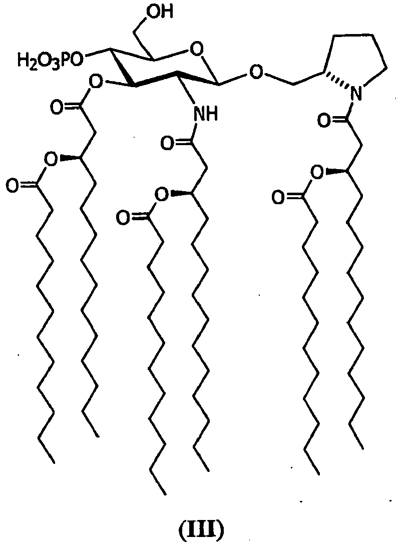 and *N*-[(*R*)-3-decanoyloxytetradecanoyl]-(*S*)-2-pynolidinylmethy 2-deoxy-4-*O*-phosphono-2-[(*R*)-3-decanoyloxytelradecanoylamino]-3-*O*-[(*R*)-3-decanoyloxytetradecanoyl]-β-D-glucopyranoside and pharmaceutically acceptable salts thereof Formula IV.

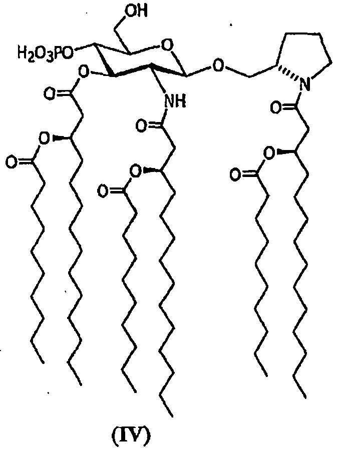

The compounds for use according to the present invention can be prepared using methods outlined in Johnson et al., Bioorg. Med. Chem. Lett. 9:2273, 1999 and PCT/WO98/50399 and references therein. In general, the synthetic methods described in the above noted reference are broadly applicable to the preparation of compounds having different acyl groups and subdivisions. For example, certain compounds useful in the present invention are described in U.S. Provisional Application No. 60/223,056 and International application PCT/US01/24284. In general, the synthetic methods described in the above-noted references described herein and other synthetic methods otherwise familiar in the art are broadly applicable to the preparation these compounds. For example, in making compounds having different acyl groups and substitutions, one of skill in the art will appreciate that the convergent methods described therein can be modified to use alternate acylating agents, or can be initiated with commercially available materials having appropriate acyl groups attached.

The term "acyl" refers to those groups derived from an aliphatic organic acid by removal of the hydroxy portion of the acid. Accordingly, acyl is meant to include, for example, acetyl, propionyl, butyryl, decanoyl, and pivaloyl.

A "(C₂-C₂₀)acyl" is an acyl group having from 2 to 20 carbons. Similarly, a (C₆-C₁₄), (C₆-C₁₂), (C₉-C₁₂), and (C₆-C₈)acyl are acyl groups having from 6 to 14 carbons, from 6 to 12 carbons, from 9 to 12 carbons, and from 6 to 8 carbons, respectively. Also included within the term "acyl" are such groups having typical substituents such as hydroxy, keto, etc.

The term 'aliphatic" by itself or as part of another substituent, means, unless otherwise stated, a straight or branched chain or cyclic, hydrocarbon moiety, including a moiety that contains both cyclical and chain elements, which may be fully saturated or mono-or polyunsaturated, having the number of carbon atoms designated (*i.e*. C₁-C₄ means one to four carbons). Examples of saturated hydrocarbon moieties include groups such as methyl, ethyl, n-propyl, isopropyl, n-butyl, t-butyl, isobutyl, sec-butyl, cyclopropyl, cyclopropylmethyl, methylene, ethylene, and n-butylene. An unsaturated alkyl group is one having one or more double and/or triple bonds. Examples of unsaturated alkyl groups include vinyl, 2-propenyl, crotyl, 1-propynyl and 2-(butadienyl).

The term "oxyaliphatic" refers to those groups having an aliphatic group attached to the remainder of the molecule through an oxygen atom.

Each of the above terms (*e.g*., "aliphatic," "acyl") are meant to include both substituted and unsubstituted forms of the indicated moiety. Preferred substituents for each type of group are provided below.

Substituents for the aliphatic groups can be a variety of groups selected from: - OR', =O, =S, =NR', N-OR', -NR'R", -SR', -halogen, -SiR'R"R"', -OC(O)R', -C(O)R', - CO₂R', -CONR'R", -OC(O)NR'R", -NR"C(O)R', -NR'-C(O)NR"R"', -NR"C(O)₂R', -NH-C(NH₂)=NH, -NR'C(NH₂)=NH, -NH-C(NH₂)=NR', -S(O)R', -S(O)₂R', -S(O)₂NR'R", -CN and -NO₂ in a number ranging from zero to (2m'+1), where m' is the total number of carbon atoms in such radical. R', R" and R'" each independently refer to hydrogen and unsubstituted (C₁-C₈)aliphatic groups. When R' and R" are attached to the same nitrogen atom, they can be combined with the nitrogen atom to form a 5-, 6-, or 7-membered ring. For example, -NR'R" is meant to include 1-pyrrolidinyl and 4-morpholinyl. From the above discussion of substituents, one of skill in the art will understand that the term "aliphatic" is meant to include groups such as haloalkyl (*e.g*., -CF₃ and -CH₂CF₃) and the like.

The terms "halo" or "halogen," by themselves or as part of another substituent, moan, unless otherwise stated, a fluorine, chlorine, bromine, or iodine atom. In compounds having halogen substituents, the halogens may be the same or different.

The term "pharmaceutically acceptable salts" is meant to include salts of the active compounds which are prepared with relatively nontoxic acids or bases, depending on the particular substituents found on the compounds described herein. When compounds for use according to the present invention contain relatively acidic functionalities, base addition salts can be obtained by addition of the desired base, either neat or in a suitable inert solvent. Examples of pharmaceutically acceptable base addition salts include sodium, potassium, calcium, ammonium, organic amino, or magnesium salt, or a similar salt. When compounds for use according to the present invention contain relatively basic functionalities, acid addition salts can be obtained by addition of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphouc, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, oxalic, maleic, malonic, benzoic, succinic, subelic, fumaric, mandelic, phthalic, benzenesulfonic, p-tolylsulfonic, citric, tartaric, methmesulforuc, and the like. Also included are salts of amino acids such as arginate and the like, and salts of organic acids like glueuronic or galactunoric acids and the like (see, for example, Berge, S.M., et al., "Pharmaceutical Salts", Journal of Pharmaceutical Science, 1977, 66, 1-19). Certain specific compounds for use according to the present invention contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

Also described herein are compounds which are in a prodrug form. Prodrugs of the compounds described herein are those compounds that readily undergo chemical changes under physiological conditions to provide the compounds for use according to the present invention. Additionally, prodrugs can be converted to compounds for use according to the present invention by chemical or biochemical methods in an ex vivo environment. For example, prodrugs can be slowly converted to the compounds for use according to the present invention when placed in a transdermal patch reservoir with a suitable enzyme or chemical reagent.

Certain compounds for use according to the present invention can exist in unsolvated forms as well as solvated forms, including hydrated forms. In general, the solvated forms are equivalent to unsolvated forms and are intended to be encompassed within the scope of the present invention. Certain compounds may exist in multiple crystalline or amorphous forms. In general, all physical forms are equivalent for the uses contemplated by the present invention and are intended to be within the scope of the present invention.

Certain compounds for use according to the present invention possess asymmetric carbon atoms (optical centers) or double bonds; the racemates, diastereomers, geometric isomers and individual isomers are all intended to be encompassed within the scope of the present invention.

The compounds for use according to the present invention may also contain unnatural proportions of atomic isotopes at one or more of the atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds, whether radioactive or not, are intended to be encompassed within the scope of the present invention.

### ILLUSTRATIVE PHARMACEUTICAL COMPOSITIONS AND THEIR DELIVERY

In another embodiment, the present invention concerns pharmaceutical compositions comprising one or more of the compounds, in combination with pharmaceutically-acceptable carriers or excipients, the compositions being for use in treating, ameliorating or substantially preventing an infectious disease, autoimmune disease or allergic condition, characterized in that the compositions are formulated in the absence of exogenous antigen and are for administration in the absence of exogenous antigen, i.e., are used in monotherapeutic applications. Such pharmaceutical compositions are useful for administration to a cell, tissue, animal or plant, either alone, or in combination with one or more other modalities of therapy. For many such embodiments, the pharmaceutical compositions of the invention will comprise one or more of the compounds described herein.

The phrase "pharmaceutically acceptable" refers to molecular entities and compositions that do not produce an allergic or similar untoward reaction when administered to a human. As used herein, "carrier" or "excipient" includes any and all solvents, dispersion media, vehicles, coatings, diluents; antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated.

Illustrative carriers for use in formulating the pharmaceutical compositions include, for example, oil-in-water or water-in-oil emulsions, aqueous compositions with or without inclusion of organic co-solvents suitable for intravenous (IV) use, liposomes or surfactant-containing vesicles, microspheres, microbeads and microsomes, powders, tablets, capsules, suppositories, aqueous suspensions, aerosols, and other carriers apparent to one of ordinary skill in the art.

In certain embodiments, the pharmaceutical compositions will comprise one or more buffers (*e.g*., neutral buffered saline or phosphate buffered saline), carbohydrates (*e.g.*, glucose, mannose, sucrose or dextrans), mannitol, proteins, polypeptides or amino acids such as glycine, antioxidants, bacteriostats, chelating agents such as EDTA or glutathione, adjuvants (*e.g*., aluminum hydroxide), solutes that render the formulation isotonic, hypotonic or weakly hypertonic with the blood of a recipient, suspending agents, thickening agents and/or preservatives.

For certain applications, aqueous formulations will be preferred, particularly those comprising an effective amount of one or more surfactants. For example, the composition can be in the form of a micellar dispersion comprising at least one suitable surfactant, *e.g*., a phospholipid surfactant. Illustrative examples of phospholipids include diacyl phosphatidyl glycerols, such as dimyristoyl phosphatidyl glycerol (DPMG), dipalmitoyl phosphatidyl glycerol (DPPG), and distearoyl phosphatidyl glycerol (DSPG), diacyl phosphatidyl cholines, such as dimyristoyl phosphatidylcholine (DPMC), dipalmitoyl phosphatidylcholine (DPPC), and distearoyl phosphatidylcholine (DSPC); diacyl phosphatidic acids, such as dimyristoyl phosphatidic acid (DPMA), dipalmitoyl phosphatidic acid (DPPA), and distearoyl phosphatidic acid (DSPA); and diacyl phosphatidyl ethanolamines such as dimyristoyl phosphatidyl ethanolamine (DPMB), dipalmitoyl phosphatidyl ethanolamine (DPPB) and distearoyl phosphatidyl ethanolamine (DSPE). Typically, a surfactant:mono-/disaccharide molar ratio in an aqueous formulation will be from about 10:1 to about 1:10, more typically from about 5:1 to about 1:5, however any effective amount of surfactant may be used in an aqueous formulation to best suit the specific objectives of interest.

As used herein, "an effective amount" is that amount which shows a response over and above the vehicle or negative controls. As discussed above, the precise dosage of the compound to be administered to a patient will depend the route of administration, the pharmaceutical composition, and the patient.

The compounds and pharmaceutical compositions of the invention can be formulated for essentially any route of administration, e.g., injection, inhalation by oral or intranasal routes, rectal, vaginal or intratracheal instillation, ingestion, or transdermal or transmucosal routes, and the like. In this way, the therapeutic effects attainable according to the the invention can be, for example, systemic, local, tissue-specific, etc., depending of the specific needs of a given application of the invention.

Illustrative formulations can be prepared and administered parenterafly, *i.e*., intraperitoneally, subcutaneously, intramuscularly or intravenously. One illustrative example of a carrier for intravenous use includes a mixture of 10% USP ethanol, 40% USP propylene glycol or polyethylene glycol 600 and the balance USP Water for Injection (WFI). Other illustrative carriers include 10% USP ethanol and USP WFI; 0.01-0.1% triethanolamine in USP WFI; or 0.01-0.2% dipalmitoyl diphosphatidylcholine in USP WFI; and 1-10% squalene or parenteral vegetable oil-in-water emulsion. Pharmaceutically acceptable parenteral solvents will generally be selected such that they provide a solution or dispersion which may be filtered through a 0.22 micron filter without removing the active ingredient.

Illustrative examples of carriers for subcutaneous or intramuscular use include phosphate buffered saline (PBS) solution, 5% dextrose in WFI and 0.01-0.1% triethanolamine in 5% dextrose or 0.9% sodium chloride in USP WFI, or a 1 to 2 or 1 to 4 mixture of 10% USP ethanol, 40% propylene glycol and the balance an acceptable isotonic solution such as 5% dextrose or 0.9% sodium chloride; or 0.01-0.2% dipalmitoyl diphosphatidylcholine in USP WFI and 1 to 10% squalene or parenteral vegetable oil-in-water emulsions.

Examples of carriers for administration via mucosal surfaces depend upon the particular route, *e.g*., oral, sublingual, intranasal, etc. When administered orally, illustrative examples include pharmaceutical grades of mannitol, starch, lactose, magnesium stearate, sodium saccharide, cellulose, magnesium carbonate and the like, with mannitol being preferred. When administered intranasally, illustrative examples include polyethylene glycol, phospholipids, glycols and glycolipids, sucrose, and/or methylcellulose, powder suspensions with or without bulking agents such as lactose and preservatives such as benzalkonium chloride, EDTA. In a particularly illustrative embodiment, the phospholipid 1,2 dipalmitoyl-sn-glycero-3-phosphocholine (DPPC) is used as an isotonic aqueous carrier at about 0.01-0.2% for intranasal administration of the compound of the subject invention at a concentration of about 0.1 to 3.0 mg/ml.

When administered by inhalation, illustrative carriers include polyethylene glycol or glycols, DPPC, methylcellulose, powdered dispersing agents, and preservatives, with polyethylene glycols and DPPC being preferred. In many instances, it will be preferred that the compounds be in a nebulized form when administration by inhalation. Illustratively, delivery may be by use of a single-use delivery device, a mist nebulizer, a breath-activated powder inhaler, an aerosol metered-dose inhaler (MDI) or any other of the numerous nebulizer delivery devices available in the art. Additionally, mist tents or direct administration through endotracheal tubes may also be used. Delivery via an intratracheal or nasopharyngeal mode will be efficacious for certain indications.

One skilled in this art will recognize that the above description is illustrative rather than exhaustive. Indeed, many additional formulations techniques and pharmaceutically-acceptable excipients and carrier solutions are well-known to those skilled in the art, as is the development of suitable dosing and treatment regimens for using the particular compositions described herein in a variety of treatment regimens.

The compounds can be evaluated in a variety of assay formats, including those described herein, to identify and select those having the characteristics best suited for a given application of the invention. For example, animal models can be used for identifying and evaluating cytokine release profiles into systemic circulation following administration of a cyclic AGP compound. In addition, various *in vitro* and *in vivo* models exist for examining changes in one or more aspects of an immune response to different antigenic components in order to identify compounds best suited for eliciting a specific immune response of interest. For example, a compound can be contacted with target cells, such as macrophages, dendritic cells or Langerhans cells *in vitro,* and elaborated cytokines can be measured. In addition, gene expression arrays can be used to identify specific pathways activated or inhibited by a particular cyclic AGP of interest.

It will be understood that, if desired, the compounds disclosed herein may be administered in combination with other therapeutic modalities, such as antimicrobial, antiviral and antifungal compounds or therapies, various DNA-based therapeutics, RNA-based therapeutics, polypeptide-based therapeutics, and/or with other immunoeffectors. In fact, essentially any other component may also be included, given that the additional component(s) do not cause a significant adverse effect upon contact with the target cells or host tissues. The compositions may thus be delivered along with various other agents as required or desired.

Illustratively, the pharmaceutical compositions of the invention can include, or be used in conjunction with, DNA encoding one or more therapeutic proteins, antisense RNAs, ribozymes or the like. The DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). In a preferred embodiment, the DNA may be introduced using a viral expression system (*e.g.*, vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad. Sci. USA 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art.

The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells. It will be apparent that a pharmaceutical composition of the invention may comprise both a polynucleotide and a protein component.

Any of a variety of additional immunostimulants may be included in the compositions of this invention. For example, cytokines, such as GM-CSF, interferons or interleukins to further modulate an immune response of interest. For example, in certain embodiments, additional components may be included in the compositions to further enhance the induction of high levels of Th1-type cytokines (*e.g.*, IFN-γ, TNFα, IL-2 and IL-12). Alternatively, or in addition, high levels of Th2-type cytokines (*e.g.*, IL-4, IL-5, IL-6 and IL-10) may be desired for certain therapeutic applications. The levels of these cytokines may be readily assessed using standard assays. For a review of the families of cytokines, see Mosmann and Coffman, Ann. Rev. Immunol. 7:145-173, 1989.

Illustrative compositions for use in induction of Th1-type cytokines include, for example, a combination of CpG-containing oligonucleotides (in which the CpG dinucleotide is unmethylated) as described, for example, in WO 96/02555, WO 99/33488 and U.S. Patent Nos. 6,008,200 and 5,856,462. Immunostimulatory DNA sequences are also described, for example, by Sato et al., Science 273:352, 1996. Other suitable immunostimulants comprise saponins, such as QS21 (Aquila Biopharmaceuticals Inc., Framingham, MA), GPI-100 (Marciani et al., Vaccine 18:3141, 2000, U.S. Patent No. 6,080,725) and related saponin deriviatives and mimetics thereof.

Other illustrative immunostimulants that can be used in conjunction with the present invention include Montanide ISA 720 (Seppic, France), SAF (Chiron, California, United States), ISCOMS (CSL), MF-59 (Chiron), the SBAS series of adjuvants (*e.g*., SBAS-2 or SBAS-4, available from SmithKline Beecham, Rixensart, Belgium), and Enhanzyn™ immunostimulant (Corixa, Hamilton, MT). Polyoxyethylene ether immunostimulants, are described in WO 99/52549A1.

The invention is further illustrated by the following non-limiting examples.

### EXAMPLES

### EXAMPLE 1

### Preparation of N-[(R)-3-Tetradecanoyloxytetradecanoyl]-(S)-2-pyrrolidinylmethyl 2-Deoxy-4-O-phosphono-2-[(R)-3-tetradecanoyloxytetradecanoylamino]-3-O-[(R)-3-tetradecanoyloxytetradecanoyl]-β-D-glucopyranoside Triethylammonium Salt; triethylammoniunm salt of the compound of Formula (II)

(1a) To as solution of 2-deoxy-4-*O*-diphenylphosphono-3-*O*-[(*R*)-3-tetradecanoyloxytetradecanoyl]-6-*O*-(2,2,2-trichloro-1,1-dimethylethoxycarbonyl)-2-(2,2,2-trichloroethoxycarbonylamino)- β-D-glucopyranosyl bromide (1.05 g, 0.81 mmol) in dry 1,2-dichloroethane (10 mL) were added 4 Å molecular sieves (0.5 g), anhydrous CaSO₄ (2.2 g, 16 mmol), and *N*-[(*R*)-3-tetradecanoyloxytetradecanoyl]-(S)-2-pyrrolidinemethanol (0.40 g, 0.75 mmol). The resulting mixture was stirred for 1 h at room temperature, treated with Hg(CN)₂ (1.02 g, 4.05 mmol), and heated to reflux for 16 h in the dark. The cooled reaction mixture was diluted with CH₂Cl₂ and filtered. The filtrate was washed with 1 N aq KI, dried (Na₂SO₄), and concentrated. Flash chromatography on silica gel (gradient elution, 15→20% EtOAc/hexanes) afforded 0.605 g (43%) of *N*-[(R)-3-tetradecanoyloxytetradecanoyl]-(*S*)-2-pyrrolidinylmethyl 2-deoxy-4-*O*-diphenylphosphono-3-*O*-[(*R*)-3-tetradecanoyloxytetradecanoyl]-6-*O*-(2,2,2-trichloro-1,1-dimethylethoxycarbonyl)-2-(2,2,2-trichloroethoxycarbonylamino)- β-D-glucopyranoside as an amorphous solid.
(1b) A solution of the compound prepared in (1a) above (0.50 g, 0.29 mmol) in AcOH (10 mL) at 60°C was treated with zinc dust (0.98 g, 15 mmol) in three equal portions over a 1-h period. The cooled reaction mixture was sonicated, filtered through a pad of Celite, and concentrated. The resulting residue was partitioned between CH₂Cl₂ and saturated aq NaHCO₃, and the layers were separated. The organic layer was dried (Na₂SO₄) and concentrated. A solution of the crude amino alcohol obtained and (*R*)-3-tetradecanoyloxytetradecanoic acid (0.155 g, 0.34 mmol) in CH₂Cl₂ (3.5 mL) was stirred with powdered 4 Å molecular sieves (0.25 g) for 0.5 h and then treated with 2-ethoxy-1-ethoxycarbonyl-1,2-dihydroquinoline (0.11 g, 0.44 mmol). The resulting mixture was stirred at room temperature for 8 h, filtered through Celite, and concentrated. Flash chromatography on silica gel with 50%. EtOAc/hexanes gave 0.355 g (68%) of *N*-[(*R*)-3-tetradecanoyloxytetradecanoyl]-(*S*)-2-pyrrolidinylmethyl 2-deoxy-4-*O*-diphenylphosphono-2-[(*R*)-3-tetradecanoyloxytetradecanoylamino]-3-*O*-[(*R*)-3-tetradecanoyloxytetradecanoyl]-β-D-glucopyranoside as a colorless syrup.
(1c) A solution of the compound prepared in (1b) above (0.300 g, 0.166 mmol) in a mixture of AcOH (1 mL) and tetrahydrofuran (9 mL) was hydrogenated in the presence of PtO₂ (0.15 g) at room temperature and 70 psig for 18 h. The reaction mixture was diluted with 2:1 CHCl₃-MeOH (50 mL) and sonicated briefly. The catalyst was collected and washed with 2:1 CHCl₃-MeOH and the combined filtrate and washings were concentrated. Flash chromatography on silica gel with CHCl₃-MeOH-H₂O-Et₃N (90:10:0.5:0.5) gave partially purified product which was dissolved in ice-cold 2:1 CHCl₃-MeOH (30 mL) and washed with ice-cold 0.1 N aq HCl (12 mL). The organic phase was filtered and lyophilized from 2% aq Et₃N (5 mL, pyrogen-free) to give 0.228 g (79%) of *N-*[(*R*)-3-tetradecanoyloxytetradecanoyl]-(*S*)-2-pyrrolidinylmethyl 2-deoxy-4-*O*-phosphono-2-[(*R*)-3-tetradecanoyloxytetradecanoylamino]-3-*O*-[(*R*)-3-tetradecanoyloxytetradecanoyl]-β-D-glucopyranoside triethylammonium salt as a colorless powder: mp 67-70 °C; IR (film) 3306, 2955, 2923, 2853, 1736, 1732, 1644, 1548, 1466, 1378, 1245, 1177, 1110, 1053, 844 cm⁻¹; ¹H NMR (CDCl₃-CD₃OD) δ 0.88 (m, 18 H), 1.0-1.2.05 (mH), 2.20-2.70 (m, 12 H), 3.06 (q, 6 H, *J* = 7.2 Hz), 3.3-325 (mH), 4.52 (d, 1 H, *J*= 8 Hz), 5.05-5.28 (m, 4 H), 7.44 (d, 1 H, *J* = 9 Hz); ¹³C NMR (CDCl₃) δ 173.3, 173.0, 170.3, 169.6, 168.6, 101.8, 100.4, 75.8, 72.5, 72.4, 70.9, 70.8, 70.3, 70.2, 69.9, 69.3, 67.9, 66.6, 56.5, 56.3, 54.5, 47.4, 45.8, 44.6, 41.4, 41.0, 39.7, 39.2, 39.0, 34.5, 34.3, 34.1, 32.0, 29.7, 29.4, 28.1, 27.3, 25.7, 25.3, 25.2, 25.1, 24.0, 22.7, 21.6, 14.1, 8.6.
   Anal. Calcd. for C₁₀₁H₁₉₄N₃O₁₇P·H₂O: C, 68.47; H, 11.15; N, 2.37; P, 1.75. Found: C, 68.79; H, 11.00; N, 2.24; P, 1.97.

### EXAMPLE 2

### Preparation of N-[(R)-3-Dodecanoyloxytetradecanoyl]-(S)-2-pyrrolidinylmethyl 2-Deoxy-4-O-phosphono-2-[(R)-3-dodecanoyloxy-tetradecanoylamino]-3-O-[(R)-3-dodecanoyloxytetradecanoyl]- β-D-glucopyranoside Triethylammonium Salt); triethylammonium salt of Formula (III)

(2a) To a solution of 2-deoxy-4-*O*-diphenylphosphono-3-*O*-[(*R*)-3-dodecanoyloxytetradecanoyl]-6-*O*-(2,2,2-trichloro-1,1-dimethylethoxycarbonyl)-2-(2,2,2-trichloroethoxycarbonylamino)-α-D-glucopyranosyl bromide (1.60 g, 1.27 mmol) in dry 1,2-dichloroethane (3.2 mL) were added 4 Å molecular sieves (0.6 g), anhydrous CaSO₄ (1.0 g, 7.3 mmol), and *N*-[(*R*)-3-dodecanoyloxytetradecanoyl]-(*S*)-2-pyrrolidinemethanol (0.58 g, 1.14 mmol). The resulting mixture was stirred for 1 h at room temperature, treated with Hg(CN)₂ (0.58 g, 2.3 mmol), and heated to reflux for 6 h in the dark. The cooled reaction mixture was diluted with CH₂Cl₂ and filtered through a bed of celite. The filtrate was washed with 1 N aq KI, dried (Na₂SO₄), and concentrated. Flash chromatography on silica gel (gradient elution, 25-->35% EtOAc/hexanes) afforded 1.72 g (82%) of *N*-[(*R*)-3-dodecanoyloxytetradecanoyl]-(*S*)-2-pyrrolidinylmethyl 2-deoxy-4-*O*-diphenylphosphono-3-*O*-[(*R*)-3-dodecanoyloxytetradecanoyl]-6-*O*-(2,2,2-trichloro-1,1-dimethylethoxycarbonyl)-2-(2,2,2-trichloroethoxycarbonylamino)- β-D-glucopyranoside as a colorless oil.
(2b) A solution of the compound prepared in (2a) above (1.58 g, 0.806 mmol) in AcOH (40 mL) at 60 °C was treated with zinc dust (2.6 g, 40 mmol) in three equal portions over a 1-h period. The cooled reaction mixture was sonicated, filtered through a pad of Celite, and concentrated. The resulting residue was partitioned between EtOAc and saturated aq NaHCO₃ and the layers separated. The organic layer was washed with brine, dried (Na₂SO₄), and concentrated to give 1.3 g of a white solid. A solution of the crude amino alcohol obtained and (*R*)-3-dodecanoyloxytetradecanoic acid (0.45 g, 1.05 mmol) in CH₂Cl₂ (20 mL) was treated with 2-ethoxy-1-ethoxycarbonyl-1,2-dihydroquinoline (0.30 g, 1.21 mmol). The resulting mixture was stirred at room temperature for 18 h and concentrated. Flash chromatography on silica gel with 40->50% EtOAc/hexanes gave 0.89 g (56%) of *N*-[(*R*)-3-dodecanoyloxytetradecanoyl]-(*S*)-2-pyrrolidinylmethyl 2-deoxy-4-*O-*diphenylphosphono-2-[(*R*)-3-dodecanoyloxytetra-decanoylamino]-3-*O*-[(*R*)-3-dodecanoyloxytetradecanoyl]-β-D-glucopyranoside as a white foam.
(2c) A solution of the compound prepared in (2b) above (0.75 g, 0.44 mmol) in a mixture of AcOH (4.5 mL) and tetrahydrofuran (45 mL) was hydrogenated in the presence of PtO₂ (0.45 g) at room temperature and 70 psig for 18 h. The reaction mixture was diluted with 2:1 CHCl₃-MeOH (35 mL) and sonicated briefly. The catalyst was collected and washed with 2:1 CHCl₃-MeOH and the combined filtrate and washings were concentrated. Flash chromatography on silica gel with CHCl₃-MeOH-H₂O-Et₃N (gradient elution; 96:4:0.3:0.3->90:10:0.5:0.5) gave partially purified product (0.51 g) which was dissolved in ice-cold 2:1 CHCl₃-MeOH (50 mL) and washed with ice-cold 0.1 N aq HCl (20 mL). The organic phase was filtered and concentrated. The white wax obtained was lyophilized from 2% aq Et₃N (70 mL, pyrogen-free) to give 0.54 g (78%) of *N*-[(*R*)-3-dodecanoyloxytetradecanoyl]-(*S*)-2-pyrrolidinylmethyl 2-deoxy-4-*O*-phosphono-2-[(*R*)-3-dodecanoyloxytetradecanoylamino]-3-*O*-[(*R*)-3-dodecanoyloxy-tetradecanoyl]-β-D-glucopyranoside triethylammonium salt as a white powder: mp 146-151 °C; IR (film) 3292, 3100, 2958, 2922, 2852, 1739, 1731, 1659, 1651, 1644, 1562, 1555, 1468, 1455, 1433, 1377, 1339, 1310, 1253, 1238, 1183, 1160, 1107, 1080, 1047, 960, 856, 722 cm⁻¹; ¹H NMR (CDCl₃-CD₃OD) δ 0.88 (m, 18 H), 1.0-2.10 (mH), 2.20-2.75 (m, 12 H), 3.04 (q, 6 H, *J* = 7.2 Hz), 3.3-4.3 (mH), 4.45 (d, 1 H, *J*= 8.5 Hz), 5.0-5.28 (m, 4 H); ¹³C NMR (CDCl₃) δ 173.9, 173.4, 173.2, 170.6, 170.1, 169.2, 101.4, 75.5, 74.0, 70.8, 70.7, 70.2, 68.5, 60.5, 56.6, 53.6, 47.4, 45.6, 40.9, 39.6, 38.8, 34.5, 34.3, 34.2, 34.1, 31.9, 29.7, 29.6, 29.5, 29.4, 29.4, 29.3, 29.2, 27.3, 25.2, 25.0, 23.6, 22.7, 21.6, 14.0, 8.3.
   MALDI-MS calculated for [M + Na]⁺ 1590.1900, found 1590.1866; Anal. Calculated for C₉₅H₁₈₂N₃O₁₇P·3H₂O: C, 66.20; H, 10.99; N, 2.44. Found: C, 66.36; H, 10.69; N, 2.15.

### EXAMPLE 3

### Preparation of N-[(R)-3-Decanoyloxytetradecanoyl]-(S)-2-pyrrolidinylmethyl 2-Deoxy-4-O-phosphono-2-[(R)-3-Decanoyloxytetradecanoylamino]-3-O-[(R)-3-Decanoyloxytetradecanoyl]- β-D-glucopyranoside Triethylammonium Salt; triethylammonium salt of Formula (IV),

(3a) To a solution of 2-deoxy-4-*O*-diphenylphosphono-3-*O*-[(*R*)-3-decanoyloxytetradecanoyl]-6-*O*-(2,2,2-trichloro-1,1-dimethylethoxycarbonyl)-2-(2,2,2-trichloroethoxycarbonylamino)-α-D-glucopyranosyl bromide (1.70 g, 1.38 mmol) in dry 1,2-dichloroethane (3.5 mL) were added 4 Å molecular sieves (0.6 g), anhydrous CaSO₄ (1.2 g, 8.8 mmol), and *N*-[(*R*)-3-decanoyloxytetradecanoyl]-(*S*)-2-pyrrolidinemethanol (0.60 g, 1.24 mmol). The resulting mixture was stirred for 1 h at room temperature, treated with Hg(CN)₂ (0.63 g, 2.5 mmol), and heated to reflux for 6 h in the dark. The cooled reaction mixture was diluted with CH₂Cl₂ and filtered through a bed of celite. The filtrate was washed with 1 N aq KI, dried (Na₂SO₄), and concentrated. Flash chromatography on silica gel (gradient elution, 25→40% EtOAc/hexanes) afforded 1.82 g (80%) of *N*-[(*R*)-3-decanoyloxytetradecanoyl]-(*S*)-2-pyrrolidinylmethyl 2-deoxy-4-*O*-diphenylphosphono-3-*O*-[(*R*)-3-decanoyloxytetradecanoyl]-6-*O*-(2,2,2-trichloro-1,1-dimethylethoxycarbonyl)-2-(2,2,2-trichloroethoxycarbonylamino)- P-D-glucopyranoside as a colorless oil.
(3b) A solution of the compound prepared in (3a) above (1.67 g, 1.02 mmol) in AcOH (50 mL) at 60 °C was treated with zinc dust (3.33 g, 51 mmol) in three equal portions over a 1-h period. The cooled reaction mixture was sonicated, filtered through a pad of Celite, and concentrated. The resulting residue was partitioned between EtOAc and saturated aq NaHCO₃ and the layers separated. The organic layer was washed with brine, dried (Na₂SO₄), and concentrated to give 1.25 g of a white solid. A solution of the crude amino alcohol obtained and (*R*)-3-decanoyloxytetradecanoic acid (0.53 g, 1.33 mmol) in CH₂Cl₂ (20 mL) was treated with 2-ethoxy-1-ethoxycarbonyl-1,2-dihydroquinoline (0.38 g, 1.53 mmol). The resulting mixture was stirred at room temperature for 18 h and concentrated. Flash chromatography on silica gel with 40->50% EtOAc/hexanes gave 1.23 g (74%) of *N*-[(*R*)-3-decanoyloxytetradecanoyl]-(*S*)-2-pyrrolidinylmethyl 2-deoxy-4-*O-*diphenylphosphono-2-[(*R*)-3-decanoyloxytetradec-anoylamino]-3-*O*-[(*R*)-3-decanoyloxytetradecanoyl]-β-D-glucopyranoside as a white foam.
(3c) A solution of the compound prepared in (3b) above (1.07 g, 0.654 mmol) in a mixture of AcOH (6.5 mL) and tetrahydrofuran (65 mL) was hydrogenated in the presence of PtO₂ (0.66 g) at room temperature and 70 psig for 18 h. The reaction mixture was diluted with 2:1 CHCl₃-MeOH (50 mL) and sonicated briefly. The catalyst was collected and washed with 2:1 CHCl₃-MeOH and the combined filtrate and washings were concentrated. The resulting waxy solid obtained was lyophilized from 2% aq triethylamine to give ~1 g of the crude triethylammonium salt as a white powder. Flash chromatography on silica gel with CHCl₃-MeOH-H₂O-Et₃N (gradient elution; 96:4:0.3:0.3->88:12:1:0.6) gave partially purified product (0.84 g) which was dissolved in ice-cold 2:1 CHCl₃-MeOH (168 mL) and washed with ice-cold 0.1 N aq HCl (67 mL). The organic phase was filtered and concentrated. The white wax obtained (~0.7 g) was lyophilized from 2% aq Et₃N (70 mL, pyrogen-free) to give 0.79 g (79%) of *N*-[(*R*)-3-decanoyloxytetradecanoyl]-(*S*)-2-pyrrolidinylmethyl 2-deoxy-4-*O*-phosphono-2-[(*R*)-3-decanoyloxytetradecanoylamino]-3-*O-*[(*R*)-3-decanoyloxytetradecanoyl]-β-D-glucopyranoside triethylammonium salt as a white powder: mp 121-122 °C; IR (film) 3287, 3093, 2961, 2913, 2850, 1745, 1738, 1732, 1716, 1666, 1660, 1651, 1644, 1635, 1565, 1556, 1538, 1470, 1455, 1434, 1416, 1378, 1337, 1311, 1248, 1184, 1104, 1081, 1021, 964, 721 cm⁻¹; ¹H NMR (CDCl₃-CD₃OD) 8 0.88 (m, 18 H), 1.0-2.05 (mH), 2.20-2.75 (m, 12 H), 3.04 (q, 6 H, *J*= 7.2 Hz), 3.3-4.3 (mH), 4.45 (d, 1 H, *J* = 8.5 Hz), 5.0-5.28 (m, 4 H); ¹³C NMR (CDCl₃) δ 173.7, 173.4, 173.2, 170.5, 170.1, 169.1, 101.4, 75.6, 74.0, 70.8, 70.2, 68.7, 60.4, 56.6, 53.8, 47.4, 45.6, 41.0, 39.6, 38.9, 34.5, 34.3, 34.2, 34.1, 31.9, 29.7, 29.6, 29.5, 29.4, 29.4, 29.3, 29.2, 27.3, 25.3, 25.0, 23.7, 22.7, 21.6, 14.1, 8.4.
   MALDI-MS calcd for [M + Na]⁺ 1506.0961, found 1506.1008; Anal. Calcd. for C₈₉H₁₇₀N₃O₁₇P: C, 67.43; H, 10.81; N, 2.65. Found: C, 67.26; H, 10.85; N, 2.47.

### EXAMPLE 4

### MURINE LISTERIA MONOCYTOGENES CHALLENGE MODEL

This example provides experiments evaluating the induction of non-specific resistance in the murine *Listeria monocytogenes* challenge model performed using the compounds prepared in examples 1, 2 and 3. Mice (5 per group) were treated intravenously with the 1 µg of a cyclic AGP or MPL solublized in 0.2% triethanolamine (TEOA). Two days later the mice were challenged intravenously with a ~10⁵ *L. monocytogenes* 10403 serotype (stock culture provided by Jory Baldridge, Washington State University, Pullman, WA.). Two days after the challenge the mice were sacrificed and the number of colony forming units (CFUs) in the spleens of individual mice were determined by plating 10-fold serial dilutions of splenic homogenates on tryptic soy agar plates. The degree of protection afforded by a given AGP or MPL was calculated by subtracting the average number of bacteria per spleen (log10 value) in the group of mice treated with a given compound, from the average number of bacteria per spleen (log10 value) in a control group that was "sham" treated with vehicle (0.2% TEOA) prior to challenge with *L. monocytogenes*.

Of the compounds tested, that of Example 3 was the most active, inducing protection that was comparable to MPL (~0.9 log 10 units). The compound of example 2 induced slightly less protection and that of example 1 was the least protective, (0.7 and 0.2 log units, respectively).

### EXAMPLE 5

### PROTECTION AGAINST LETHAL INFLUENZA CHALLENGE BY PROPHYLACTIC ADMINISTRATION OF CYCLIC AGPs

This example provides experiments evaluating protection against a lethal challenge with influenza in cyclic AGP-treated mice. BALB/c mice (10 mice per group) were treated intranasally with 20 µg of the compounds of examples 1, 2 and 3, or with MPL, 48 hours prior to an lethal intranasal challenge of Influenza A/HK/68 (5 LD50). Protection was judged by survival, observations of clinical symptoms (ruffled fur, hunched posture and labored breathing), and prevention of weight loss for 21 days following the challenge.

As was seen in the Listeria model, the compounds of examples 2 and 3 provided enhanced protection when compared with the vehicle control. Mice treated with the compound of example 3 had a 60% survival rate; those treated with the compound of example 2 had a 40% survival rate, and those with MPL, a 30% survival rate. No mice tretaed with the compound of example 1 survived. These data indicate that the compound of examle 3 provided superior protection, followed by those of examples 2 and 1.

## Claims

1. One or more compounds for use in treating, ameliorating or substantially preventing an infectious disease, autoimmune disease or allergic condition, the one or more compounds having the formula: 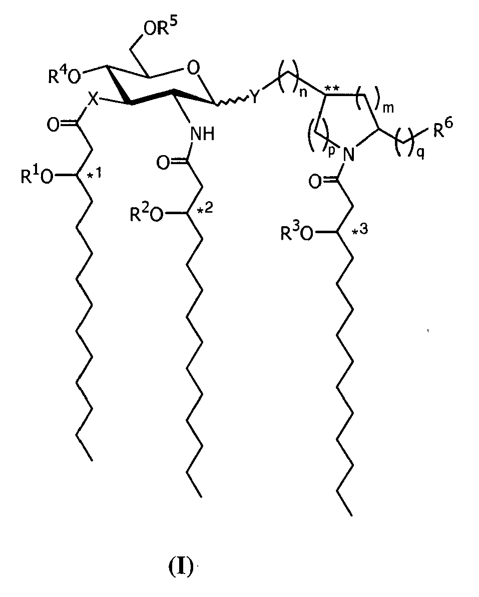 and pharmaceutically acceptable salts thereof, wherein X is a member selected from the group consisting of -O- and -NH-;
Y is a member selected from the group consisting of -O- and -S-;
R¹, R² and R³ are each members independently selected from the group consisting of (C₂-C₂₀)acyl;
R⁴ is a member selected from the group consisting of -H and -PO₃R⁷R⁸, wherein R⁷ and R⁸ are each members independently selected from the group consisting of -H and (C₁-C₄)aliphatic groups;
R⁵ is a member selected from the group consisting of -H, -CH₃ and -PO₃R⁹R¹⁰, wherein R⁹ and R¹⁰ are each members independently selected from the group consisting of -H and (C₁-C₄)aliphatic groups;
R⁶ is selected from H, OH, (C₁-C₄)oxyaliphatic groups, -PO₃R¹¹R¹², - OPO₃R¹¹R¹², -SO₃R¹¹, -OSO₃R¹¹, -NR¹¹R¹², -SR¹¹, -CN, -NO₂, -CHO, -CO₂R¹¹, and -CONR¹¹R¹², wherein R¹¹ and R¹² are each independently selected from H and (C₁-C₄)aliphatic groups, with the provisos that one of R⁴ and R⁵ is a phosphorus-containing group and that when R⁴ is -PO₃R⁷R⁸, R⁵ is other than -PO₃R⁹R¹⁰;
wherein "*¹", "*²", "*³" and "**" represent chiral centers;
wherein *n, m, p* and *q* are each independently an integer from 0 to 6, with the proviso that the sum of *p* and m is from 0 to 6,
and wherein the hexapyranoside may be in another configuration than the gluco configuration represented in the formula, selected from allo, altro, manno, gulo, ido, galacto and talo;
**characterised in that** the one or more compounds are administered in the absence of exogenous antigen.

2. One or more compounds as claimed in claim 1 for use according to claim 1, wherein R⁷ and R⁸ are -H.

3. One or more compounds as claimed in claim 1 or 2 for use according to claim 1 or claim 2, wherein X and Y are -O-, R⁴ is PO₃R⁷R⁸, R⁵ and R⁶ are H, and *n, m, p*, and *q* are integers from 0 to 3.

4. One or more compounds as claimed in claim 3 for use according to claim 3, wherein *n, m, p*, and q are from 0 to 2.

5. One or more compounds as claimed in claim 3 for use according to claim 3, wherein *n* is 1, *m* is 2, and *p* and *q* are 0.

6. One or more compounds as claimed in any preceding claim for use according to any preceding claim wherein R₁, R₂ and R₃ are each C₆-C₁₄ acyl.

7. One or more compounds as claimed in any of claims 1 to 5 for use according to any of claims 1 to 5 wherein R₁, R₂ and R₃ are each C₆-C₁₂ acyl.

8. One or more compounds as claimed in any of claims 1 to 7 for use according to any of claims 1 to 5 wherein R₁, R₂, and R₃ are each decanoyl residues.

9. One or more compounds as claimed in any of claims 1 to 7 for use according to any of claims 1 to 5 wherein R₁, R₂, and R₃ are each dodecanoyl residues.

10. One or more compounds as claimed in any of claims 1 to 6 for use according to any of claims 1 to 5 wherein R₁, R₂, and R₃ are each tetradecanoyl residues.

11. One or more compounds as claimed in any preceding claim for use according to any preceding claim, wherein *1, *2, and *3 are in the *R* configuration.

12. One or more compounds as claimed in any preceding claim for use according to any preceding claim, wherein Y is in the equatorial position.

13. One or more compounds as claimed in any preceding claim for use according to any preceding claim, wherein ** is in the *S* configuration.

14. One or more compounds as claimed in any of claims 1 to 10 for use according to any of claims 1 to 10, wherein *¹, *², and *³ are in the *R* configuration, wherein Y is in the equatorial position, and wherein ** is in the *S* configuration.

15. One or more compounds as claimed in any preceding claim for use according to any preceding claim wherein said infectious disease is caused by a bacterium, a virus, a parasite, or a fungus.

16. One or more compounds as claimed in claim 15 for use according to claim 15, wherein said bacterium is a gram negative bacterium, or a gram positive bacterium.

17. One or more compounds as claimed in claim 15 for use according to claim 15, wherein the infectious disease is caused by a bacterium selected from the group consisting of *Pseudomonas, Escherichia, Klebsiella, Enterobacter, Proteus, Serratia, Candida, Bacillus,* and *Staphylococcus.*

18. One or more compounds as claimed in claim 17 for use according to claim 17, wherein the infectious disease is pneumonia.

19. One or more compounds as claimed in claim 18 for use according to claim 18, wherein said pneumonia is nosocomial pneumonia.

20. One or more compounds as claimed in claim 19 for use according to claim 19, wherein said pneumonia is in an HIV-positive patient.

21. One or more compounds as claimed in any of claims 1 to 14 for use according to any of claims 1 to 14 wherein said infectious disease is a chronic infection.

22. One or more compounds as claimed in claim 21 for use according to claim 21, wherein said chronic infection comprises chronic hepatitis, human papillomavirus, oral or vaginal candidiasis, periodontal disease or chronic rhinosinusitis due to fungal colonization.

23. One or more compounds as claimed in any of claims 1 to 14 for use according to any of claims 1 to 14 wherein said allergic condition is selected from the group consisting of asthma, atopic dermatitis, seasonal allergic disorder and chronic rhinosinusitis.

24. One or more compounds as claimed in any of claims 1 to 14 for use according to any of claims 1 to 14 wherein said autoimmune disease is selected from the group consisting of inflammatory bowel disease, rheumatoid arthritis, chronic arthritis, multiple sclerosis and psoriasis.

25. One or more compounds as claimed in any preceding claim for use according to any preceding claim wherein the one or more compounds is for use in administration by a route selected from the group consisting of parenteral, oral, intravenous, infusion, intranasal, inhalation, transdermal and transmucosal.

26. One or more compounds as claimed in any of claims 1 to 14 for use according to any of claims 1 to 14 wherein said use is in the prophylactic treatment of infectious diseases.

27. One or more compounds as claimed in claim 26 for use according to claim 26, wherein the infectious disease is caused by a bacterium, a virus, a parasite, or a fungus.

28. One or more compounds as claimed in claim 27 for use according to claim 27, wherein said bacterium is a gram negative bacterium, or a gram positive bacterium.

29. One or more compounds as claimed in claim 27 for use according to claim 27, wherein the infectious disease is caused by a bacterium selected from the group consisting of *Pseudomonas, Escherichia, Klebsiella, Enterobacter, Proteus, Serratia, Candida, Bacillus,* and *Staphylococcus.*

30. One or more compounds as claimed in claim 29 for use according to claim 29, wherein the infectious disease is pneumonia.

31. One or more compounds as claimed in claim 30 for use according to claim 30, wherein said pneumonia is nosocomial pneumonia.

32. A cyclic AGP for use in treating, ameliorating or substantially preventing an infectious disease, autoimmune disease or allergic condition, wherein said cyclic AGP is *N*-[(*R*)-3-dodecanoyloxytetradecanoyl]-(*S*)-2-pyrrolidinylmethyl-2-deoxy-4-*O-*phosphono-2-[(*R*)-3-dodecanoyloxy-tetradecanoylamino]-3-O-[(*R*)-3-dodecanoyloxytetradecanoyl]- β-D-glucopyranoside or a pharmaceutically acceptable salt thereof; or *N*-[(*R*)-3-decanoyloxytetradecanoyl]-(*S*)-2-pyrrolidinylmethyl-2-deoxy-4-*O-*phosphono-2-[(*R*)-3-decanoyloxytetradecanoylamino]-3-*O*-[(*R*)-3-decanoyloxytetradecanoyl]- β-D-glucopyranoside or a pharmaceutically acceptable salt thereof;
**characterised in that**
said cyclic AGP is administered in the absence of exogenous antigen.

33. A cyclic AGP as claimed in claim 32 for use according to claim 32 wherein the use comprises a monotherapy without administration of an exogenous antigen.

34. A pharmaceutical composition comprising one or more compounds having a structure as defined in any one of claims 1 to 14 or claim 32 in combination with pharmaceutically-acceptable carriers or excipients, the composition being for use in treating, ameliorating or substantially preventing an infectious disease, autoimmune disease or allergic condition;
**characterised in that**
said composition is formulated in the absence of exogenous antigen and is for administration in the absence of exogenous antigen.

35. A pharmaceutical composition according to claim 34 wherein said use is as defined in any of claims 15 to 31.

## Patentansprüche

1. Eine oder mehrere Verbindungen zur Verwendung beim Behandeln, Verbessern oder im wesentlichen Verhindern einer infektiösen Erkrankung, Autoimmunerkrankung oder eines allergischen Zustands, wobei die eine oder mehreren Verbindungen die Formel aufweisen: 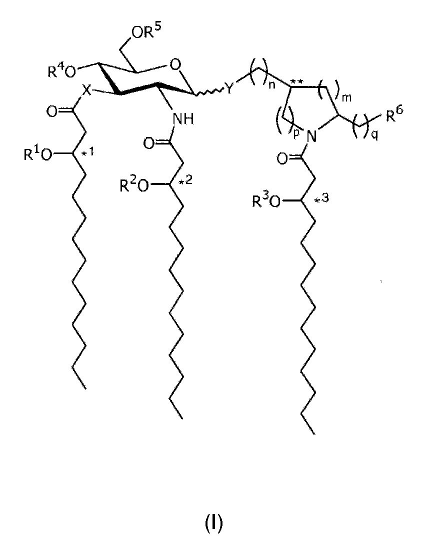 und pharmazeutisch annehmbare Salze davon, worin X ein Mitglied ist, gewählt aus der Gruppe bestehend aus -O- und -NH-;
Y ein Mitglied ist, gewählt aus der Gruppe bestehend aus -O- und -S-;
R¹, R² und R³ jeweils Mitglieder sind, unabhängig voneinander gewählt aus der Gruppe bestehend aus (C₂-C₂₀)-Acyl;
R⁴ ein Mitglied ist, gewählt aus der Gruppe bestehend aus -H und -PO₃R⁷R⁸, worin R⁷ und R⁸ jeweils Mitglieder sind, unabhängig voneinander gewählt aus der Gruppe bestehend aus -H und (C₁-C₄)-aliphatische Gruppen;
R⁵ ein Mitglied ist, gewählt aus der Gruppe bestehend aus -H, -CH₃ und -PO₃R⁹R¹⁰, worin R⁹ und R¹⁰ jeweils Mitglieder sind, unabhängig voneinander gewählt aus der Gruppe bestehend aus -H und (C₁-C₄)-aliphatische Gruppen;
R⁶ gewählt ist aus H, OH, (C₁-C₄)-oxyaliphatische Gruppen, -PO₃R¹¹R¹², -OPO₃R¹¹R¹², -SO₃R¹¹, -OSO₃R¹¹, -NR¹¹R¹², -SR¹¹, -CN, -NO₂, -CHO, -CO₂R¹¹ und -CONR¹¹R¹², worin R¹¹ und R¹² jeweils unabhängig voneinander gewählt sind aus H und (C₁-C₄)-aliphatische Gruppen, mit der Maßgabe, dass eines von R⁴ und R⁵ eine phosphorhaltige Gruppe ist und dass wenn R⁴ -PO₃R⁷R⁸ ist, R⁵ eine andere Gruppe als -PO₃R⁹R¹⁰ ist;
worin "*¹", "*²", "*³" und "**" Chiralitätszentren darstellen;
worin *n,* m, *p* und q jeweils unabhängig voneinander eine ganze Zahl von 0 bis 6 sind, mit der Maßgabe, dass die Summe von *p* und *m* eine Zahl von 0 bis 6 ist,
und worin das Hexapyranosid in einer anderen Konfiguration vorliegen kann als die in der Formel wiedergegebene Gluco-Konfiguration, ausgewählt aus Allo, Altro, Manno, Gulo, Ido, Galacto und Talo;
**dadurch gekennzeichnet, dass** die eine oder mehreren Verbindungen in Abwesenheit eines exogenen Antigens verabreicht werden.

2. Eine oder mehrere Verbindungen nach Anspruch 1 zur Verwendung nach Anspruch 1, worin R⁷ und R⁸ -H sind.

3. Eine oder mehrere Verbindungen nach Anspruch 1 oder 2 zur Verwendung nach Anspruch 1 oder 2, worin X und Y -O- sind, R⁴ PO₃R⁷R⁸ ist, R⁵ und R⁶ H sind und *n*, *m*, *p* und *q* ganze Zahlen von 0 bis 3 sind.

4. Eine oder mehrere Verbindungen nach Anspruch 3 zur Verwendung nach Anspruch 3, worin *n*, *m*, *p* und *q* Zahlen von 0 bis 2 sind.

5. Eine oder mehrere Verbindungen nach Anspruch 3 zur Verwendung nach Anspruch 3, worin *n* 1 ist, *m* 2 ist und *p* und *q* 0 sind.

6. Eine oder mehrere Verbindungen nach einem vorangehenden Anspruch zur Verwendung nach einem vorangehenden Anspruch, worin R¹, R² und R³ jeweils C₆-C₁₄-Acyl sind.

7. Eine oder mehrere Verbindungen nach einem der Ansprüche 1 bis 5 zur Verwendung nach einem der Ansprüche 1 bis 5, worin R¹, R² und R³ jeweils C₆-C₁₂-Acyl sind.

8. Eine oder mehrere Verbindungen nach einem der Ansprüche 1 bis 7 zur Verwendung nach einem der Ansprüche 1 bis 5, worin R¹, R² und R³ jeweils Decanoyl-Reste sind.

9. Eine oder mehrere Verbindungen nach einem der Ansprüche 1 bis 7 zur Verwendung nach einem der Ansprüche 1 bis 5, worin R¹, R² und R³ jeweils Dodecanoyl-Reste sind.

10. Eine oder mehrere Verbindungen nach einem der Ansprüche 1 bis 6 zur Verwendung nach einem der Ansprüche 1 bis 5, worin R¹, R² und R³ jeweils Tetradecanoyl-Reste sind.

11. Eine oder mehrere Verbindungen nach einem vorangehenden Anspruch zur Verwendung nach einem vorangehenden Anspruch, worin *¹, *² und *³ in der R-Konfiguration sind.

12. Eine oder mehrere Verbindungen nach einem vorangehenden Anspruch zur Verwendung nach einem vorangehenden Anspruch, worin Y in der äquatorialen Position ist.

13. Eine oder mehrere Verbindungen nach einem vorangehenden Anspruch zur Verwendung nach einem vorangehenden Anspruch, worin ** in der S-Konfiguration ist.

14. Eine oder mehrere Verbindungen nach einem der Ansprüche 1 bis 10 zur Verwendung nach einem der Ansprüche 1 bis 10, worin *¹, *² und *³ in der R-Konfiguration sind, worin Y in der äquatorialen Position ist und worin ** in der S-Konfiguration ist.

15. Eine oder mehrere Verbindungen nach einem vorangehenden Anspruch zur Verwendung nach einem vorangehenden Anspruch, worin die infektiöse Erkrankung durch ein Bakterium, einen Virus, einen Parasiten oder einen Pilz verursacht ist.

16. Eine oder mehrere Verbindungen nach Anspruch 15 zur Verwendung nach Anspruch 15, worin das Bakterium ein Gram-negatives Bakterium oder ein Gram-positives Bakterium ist.

17. Eine oder mehrere Verbindungen nach Anspruch 15 zur Verwendung nach Anspruch 15, worin die infektiöse Erkrankung verursacht ist durch ein Bakterium, gewählt aus der Gruppe bestehend aus *Pseudomonas, Escherichia, Klebsiella, Enterobacter, Proteus, Serratia, Candida, Bacillus* und *Staphylococcus.*

18. Eine oder mehrere Verbindungen nach Anspruch 17 zur Verwendung nach Anspruch 17, worin die infektiöse Erkrankung Pneumonie ist.

19. Eine oder mehrere Verbindungen nach Anspruch 18 zur Verwendung nach Anspruch 18, worin die Pneumonie nosokomiale Pneumonie ist.

20. Eine oder mehrere Verbindungen nach Anspruch 19 zur Verwendung nach Anspruch 19, worin die Pneumonie bei einem HIV-positiven Patienten vorliegt.

21. Eine oder mehrere Verbindungen nach einem der Ansprüche 1 bis 14 zur Verwendung nach einem der Ansprüche 1 bis 14, worin die infektiöse Erkrankung eine chronische Infektion ist.

22. Eine oder mehrere Verbindungen nach Anspruch 21 zur Verwendung nach Anspruch 21, worin die chronische Infektion chronische Hepatitis, humanes Papillomavirus, orale oder vaginale Candidose, Parodontalerkrankung oder chronische Rhinosinusitis durch Pilzbefall umfasst.

23. Eine oder mehrere Verbindungen nach einem der Ansprüche 1 bis 14 zur Verwendung nach einem der Ansprüche 1 bis 14, worin der allergische Zustand gewählt ist aus der Gruppe bestehend aus Asthma, atopische Dermatitis, saisonale allergische Erkrankung und chronische Rhinosinusitis.

24. Eine oder mehrere Verbindungen nach einem der Ansprüche 1 bis 14 zur Verwendung nach einem der Ansprüche 1 bis 14, worin die Autoimmunerkrankung gewählt ist aus der Gruppe bestehend aus entzündliche Darmerkrankung, rheumatoide Arthritis, chronische Arthritis, Multiple Sklerose und Psoriasis.

25. Eine oder mehrere Verbindungen nach einem vorangehenden Anspruch zur Verwendung nach einem vorangehenden Anspruch, worin die eine oder mehreren Verbindungen zur Verwendung in der Verabreichung auf einer Route ist, gewählt aus der Gruppe bestehend aus parenteral, oral, intravenös, Infusion, intranasal, Inhalation, transdermal und transmukosal.

26. Eine oder mehrere Verbindungen nach einem der Ansprüche 1 bis 14 zur Verwendung nach einem der Ansprüche 1 bis 14, worin die Verwendung in der prophylaktischen Behandlung einer infektiösen Erkrankung ist.

27. Eine oder mehrere Verbindungen nach Anspruch 26 zur Verwendung nach Anspruch 26, worin die infektiöse Erkrankung durch ein Bakterium, einen Virus, einen Parasiten oder einen Pilz verursacht ist.

28. Eine oder mehrere Verbindungen nach Anspruch 27 zur Verwendung nach Anspruch 27, worin das Bakterium ein Gram-negatives Bakterium oder ein Gram-positives Bakterium ist.

29. Eine oder mehrere Verbindungen nach Anspruch 27 zur Verwendung nach Anspruch 27, worin die infektiöse Erkrankung verursacht ist durch ein Bakterium, gewählt aus der Gruppe bestehend aus *Pseudomonas, Escherichia, Klebsiella, Enterobacter, Proteus, Serratia, Candida, Bacillus* und *Staphylococcus.*

30. Eine oder mehrere Verbindungen nach Anspruch 29 zur Verwendung nach Anspruch 29, worin die infektiöse Erkrankung Pneumonie ist.

31. Eine oder mehrere Verbindungen nach Anspruch 30 zur Verwendung nach Anspruch 30, worin die Pneumonie nosokomiale Pneumonie ist.

32. Cyclisches AGP zur Verwendung beim Behandeln, Verbessern oder im wesentlichen Verhindern einer infektiösen Erkrankung, Autoimmunerkrankung oder eines allergischen Zustands, worin das cyclische AGP N-[(R)-3-Dodecanoyloxytetradecanoyl]-(S)-2-pyrrolidinylmethyl-2-deoxy-4-O-phosphono-2-[(R)-3-dodecanoyloxy-tetradecanoylamino]-3-O-[(R)-3-dodecanoyloxytetradecanoyl]-β-D-glucopyranosid oder ein pharmazeutisch annehmbares Salz davon oder N-[(R)-3-Decanoyloxytetradecanoyl]-(S)-2-pyrrolidinylmethyl-2-deoxy-4-O-phosphono-2-[(R)-3-decanoyloxytetradecanoylamino]-3-O-[(R)-3-decanoyloxytetradecanoyl]-β-D-glucopyranosid oder ein pharmazeutisch annehmbares Salz davon, **dadurch gekennzeichnet, dass** das cyclische AGP in Abwesenheit eines exogenen Antigens verabreicht wird.

33. Cyclisches AGP nach Anspruch 32 zur Verwendung nach Anspruch 32, worin die Verwendung eine Monotherapie ohne Verabreichung eines exogenen Antigens umfasst.

34. Pharmazeutische Zusammensetzung, umfassend eine oder mehrere Verbindungen mit einer Struktur wie in einem der Ansprüche 1 bis 14 oder Anspruch 32 definiert, in Kombination mit pharmazeutisch annehmbaren Trägern oder Hilfsstoffen, wobei die Zusammensetzung zur Verwendung beim Behandeln, Verbessern oder im wesentlichen Verhindern einer infektiösen Erkrankung, Autoimmunerkrankung oder eines allergischen Zustands ist, **dadurch gekennzeichnet, dass** die Zusammensetzung in Abwesenheit eines exogenen Antigens formuliert ist und zur Verabreichung in Abwesenheit eines exogenen Antigens ist.

35. Pharmazeutische Zusammensetzung nach Anspruch 34, worin die Verwendung wie in einem der Ansprüche 15 bis 31 definiert ist.

## Revendications

1. Un ou plusieurs composés pour une utilisation dans le traitement, l'amélioration ou la prévention de manière substantielle d'une maladie infectieuse, d'une maladie auto-immune ou d'une affection allergique, les un ou plusieurs composés ayant la formule : 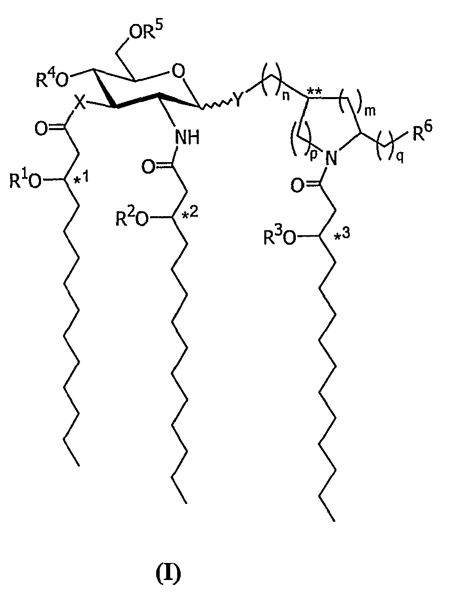 et des sels pharmaceutiquement acceptables de ceux-ci, où X est un membre choisi dans le groupe constitué par -O- et -NH-;
Y est un membre choisi dans le groupe constitué par -O- et -S- ;
R¹, R² et R³ sont chacun des membres choisis indépendamment dans le groupe constitué par des groupes acyle en C₂ à C₂₀;
R⁴ est un membre choisi dans le groupe constitué par -H et - PO₃R⁷R⁸, où R⁷ et R⁸ sont chacun des membres choisis indépendamment dans le groupe constitué par -H et des groupes aliphatiques en C₁ à C₄ ;
R⁵ est un membre choisi dans le groupe constitué par -H, -CH₃ et - PO₃R⁹R¹⁰, où R⁹ et R¹⁰ sont chacun des membres choisis indépendamment dans le groupe constitué par -H et des groupes aliphatiques en C₁ à C₄ ;
R⁶ est choisi parmi H, les groupes OH, oxyaliphatiques en C₁ à C₄, - PO₃R¹¹R¹², -OPO₃R¹¹R¹², -SO₃R¹¹, -OSO₃R¹¹, -NR¹¹R¹², -SR¹¹, -CN, - NO₂, -CHO, -CO₂R¹¹ et -CONR¹¹R¹², où R¹¹ et R¹² sont choisis chacun indépendamment parmi H et des groupes aliphatiques en C₁ à C₄, à condition que l'un de R⁴ et R⁵ soit un groupe contenant du phosphore et que lorsque R⁴ représente -PO₃R⁷R⁸, R⁵ soit différent de -PO₃R⁹R¹⁰;
où « *¹ », « *² », « *³ » est « ** » représentent des centres chiraux ;
où *m*, *m*, *p* et *q* représentent chacun indépendamment un nombre entier de 0 à 6, à condition que la somme de *p* et *m* vaille de 0 à 6,
et où l'hexapyranoside peut être dans une autre configuration que la configuration gluco représentée sur la formule, choisie parmi allo, altro, manno, gulo, ido, galacto et talo ;
**caractérisés en ce que** les un ou plusieurs composés sont administrés en l'absence d'antigène exogène.

2. Un ou plusieurs composés selon la revendication 1, pour une utilisation selon la revendication 1, dans lesquels R⁷ et R⁸ représentent -H.

3. Un ou plusieurs composés selon la revendication 1 ou 2 pour une utilisation selon la revendication 1 ou la revendication 2, dans lesquels X et Y représentent -O-, R⁴ représente PO₃R⁷R⁸, R⁵ et R⁶ représentent H, et *n*, *m*, *p* et *q* sont des nombres entiers de 0 à 3.

4. Un ou plusieurs composés selon la revendication 3 pour une utilisation selon la revendication 3, dans lesquels *n*, *m*, *p* et *q* valent de 0 à 2.

5. Un ou plusieurs composés selon la revendication 3 pour une utilisation selon la revendication 3, dans lesquels *n* vaut 1, *m* vaut 2, et *p* et *q* valent 0.

6. Un ou plusieurs composés selon l'une quelconque des revendications précédentes pour une utilisation selon l'une quelconque des revendications précédentes, dans lesquels R¹, R² et R³ représentent chacun des groupes acyle en C₆ à C₁₄.

7. Un ou plusieurs composés selon l'une quelconque des revendications 1 à 5 pour une utilisation selon l'une quelconque des revendications 1 à 5, dans lesquels R¹, R² et R³ représentent chacun des groupes acyle en C₆ à C₁₂.

8. Un ou plusieurs composés selon l'une quelconque des revendications 1 à 7 pour une utilisation selon l'une quelconque des revendications 1 à 5, dans lesquels R¹, R² et R³ représentent chacun des résidus décanoyle.

9. Un ou plusieurs composés selon l'une quelconque des revendications 1 à 7 pour une utilisation selon l'une quelconque des revendications 1 à 5, dans lesquels R¹, R² et R³ représentent chacun des résidus dodécanoyle.

10. Un ou plusieurs composés selon l'une quelconque des revendications 1 à 6 pour une utilisation selon l'une quelconque des revendications 1 à 5, dans lesquels R¹, R² et R³ représentent chacun des résidus tétradécanoyle.

11. Un ou plusieurs composés selon l'une quelconque des revendications précédentes pour une utilisation selon l'une quelconque des revendications précédentes, dans lesquels *¹, *² et *³ sont dans la configuration *R*.

12. Un ou plusieurs composés selon l'une quelconque des revendications précédentes pour une utilisation selon l'une quelconque des revendications précédentes, dans lesquels Y est en position équatoriale.

13. Un ou plusieurs composés selon l'une quelconque des revendications précédentes pour une utilisation selon l'une quelconque des revendications précédentes, dans lesquels ** est dans la configuration *S*.

14. Un ou plusieurs composés selon l'une quelconque des revendications 1 à 10 pour une utilisation selon l'une quelconque des revendications 1 à 10, dans lesquels *¹, *² et *³ sont dans la configuration *R*, dans lesquels Y est en position équatoriale, et dans lesquels ** est dans la configuration *S*.

15. Un ou plusieurs composés selon l'une quelconque des revendications précédentes pour une utilisation selon l'une quelconque des revendications précédentes, où ladite maladie infectieuse est provoquée par une bactérie, un virus, un parasite ou un champignon.

16. Un ou plusieurs composés selon la revendication 15 pour une utilisation selon la revendication 15, où ladite bactérie est une bactérie Gram négatif, ou une bactérie Gram positif.

17. Un ou plusieurs composés selon la revendication 15 pour une utilisation selon la revendication 15, où la maladie infectieuse est provoquée par une bactérie choisie dans le groupe constitué par *Pseudomonas, Escherichia, Klebsiella, Enterobacter, Proteus, Serratia, Candida, Bacillus* et *Staphylococcus.*

18. Un ou plusieurs composés selon la revendication 17 pour une utilisation selon la revendication 17, où la maladie infectieuse est une pneumonie.

19. Un ou plusieurs composés selon la revendication 18 pour une utilisation selon la revendication 18, où ladite pneumonie est une pneumonie nosocomiale.

20. Un ou plusieurs composés selon la revendication 19 pour une utilisation selon la revendication 19, où ladite pneumonie est chez un patient positif pour le VIH.

21. Un ou plusieurs composés selon l'une quelconque des revendications 1 à 14 pour une utilisation selon l'une quelconque des revendications 1 à 14, où ladite maladie infectieuse est une infection chronique.

22. Un ou plusieurs composés selon la revendication 21 pour une utilisation selon la revendication 21, où ladite affection chronique comprend une hépatite chronique, un papillomavirus humain, une candidose orale ou vaginale, une maladie parodontale ou une rhinosinusite chronique due à une colonisation fongique.

23. Un ou plusieurs composés selon l'une quelconque des revendications 1 à 14 pour une utilisation selon l'une quelconque des revendications 1 à 14, où ladite affection allergique est choisie dans le groupe constitué par l'asthme, la dermite atopique, un trouble allergique saisonnier et une rhinosinusite chronique.

24. Un ou plusieurs composés selon l'une quelconque des revendications 1 à 14 pour une utilisation selon l'une quelconque des revendications 1 à 14, où ladite maladie auto-immune est choisie dans le groupe constitué par une maladie intestinale inflammatoire, la polyarthrite rhumatoïde, une arthrite chronique, la sclérose en plaques et le psoriasis.

25. Un ou plusieurs composés selon l'une quelconque des revendications précédentes pour une utilisation selon l'une quelconque des revendications précédentes, où les un ou plusieurs composés sont pour une utilisation dans l'administration par une voie choisie dans le groupe constitué par les voies parentérale, orale, intraveineuse, par perfusion, intranasale, par inhalation, transdermique et transmucosale.

26. Un ou plusieurs composés selon l'une quelconque des revendications 1 à 14 pour une utilisation selon l'une quelconque des revendications 1 à 14, où ladite utilisation est dans le traitement prophylactique de maladies infectieuses.

27. Un ou plusieurs composés selon la revendication 26 pour une utilisation selon la revendication 26, où la maladie infectieuse est provoquée par une bactérie, un virus, un parasite ou un champignon.

28. Un ou plusieurs composés selon la revendication 27 pour une utilisation selon la revendication 27, où ladite bactérie est une bactérie Gram négatif ou une bactérie Gram positif.

29. Un ou plusieurs composés selon la revendication 27 pour une utilisation selon la revendication 27, où la maladie infectieuse est provoquée par une bactérie choisie dans le groupe constitué par *Pseudomonas, Escherichia, Klebsiella, Enterobacter, Proteus, Serratia, Candida, Bacillus* et *Staphylococcus*.

30. Un ou plusieurs composés selon la revendication 29 pour une utilisation selon la revendication 29, où la maladie infectieuse est une pneumonie.

31. Un ou plusieurs composés selon la revendication 30 pour une utilisation selon la revendication 30, où ladite pneumonie est une pneumonie nosocomiale.

32. AGP cyclique pour une utilisation dans le traitement, l'amélioration ou la prévention de manière substantielle d'une maladie infectieuse, d'une maladie auto-immune ou d'une affection allergique, où ledit AGP cyclique est le *N*-[(*R*)-3-dodécanoyloxytétradécanoyl]-(*S*)-2-pyrrolidinylméthyl-2-désoxy-4-*O*-phosphono-2-[(*R*)-3-dodécanoyloxytétradécanoylamino]-3-*O*-[(*R*)-3-dodécanoyloxytétradécanoyl]-β-D-glucopyranoside ou un sel pharmaceutiquement acceptable de celui-ci ; ou le *N*-[(*R*)-3-décanoyloxytétradécanoyl]-(*S*)-2-pyrrolidinylméthyl-2-désoxy-4-*O-*phosphono-2-[(*R*)-3-décanoyloxytétradécanoylamino]-3-*O*-[(*R*)-3-décanoyloxytétradécanoyl]-β-D-glucopyranoside ou un sel pharmaceutiquement acceptable de celui-ci ;
**caractérisé en ce que**
ledit AGP cyclique est administré en l'absence d'antigène exogène.

33. AGP cyclique selon la revendication 32 pour une utilisation selon la revendication 32, où l'utilisation comprend une monothérapie sans administration d'un antigène exogène.

34. Composition pharmaceutique comprenant un ou plusieurs composés ayant une structure telle que définie dans l'une quelconque des revendications 1 à 14 ou la revendication 32 en combinaison avec des supports ou des excipients pharmaceutiquement acceptables, la composition étant pour une utilisation dans le traitement, l'amélioration ou la prévention de manière substantielle d'une maladie infectieuse, d'une maladie auto-immune ou d'une affection allergique ;
**caractérisée en ce que**
ladite composition est formulée en l'absence d'antigène exogène et est pour une administration en l'absence d'antigène exogène.

35. Composition pharmaceutique selon la revendication 34, où ladite utilisation est telle que définie dans l'une quelconque des revendications 15 à 31.
